# EUROPÄISCHE PATENTSCHRIFT

(11) **EP 1 076 638 B1**
(45) Veröffentlichungstag und Bekanntmachung des Hinweises auf die Patenterteilung: **18.06.2003**
(21) Anmeldenummer: 99917975.7
(22) Anmeldetag: 13.04.1999
(51) Int. Cl.: C07C 7/08

(54) **VERFAHREN ZUM TRENNEN EINES C4-KOHLENWASSERSTOFFGEMISCHES**
METHOD FOR SEPARATING A C 4? HYDROCARBON MIXTURE
PROCEDE POUR SEPARER UN MELANGE D'HYDROCARBURES C 4?

(30) Priorität: 27.04.1998 DE 19818810
(43) Veröffentlichungstag der Anmeldung: 21.02.2001
(73) Patentinhaber: BASF AKTIENGESELLSCHAFT, 67056 Ludwigshafen (DE)
(72) Erfinder: KINDLER, Klaus, D-67376 Harthausen (DE); PUHL, Hubert, D-67063 Ludwigshafen (DE)
(86) Internationale Anmeldenummer: EP9902470
(87) Internationale Veröffentlichungsnummer: WO99055647

(56) Entgegenhaltungen:
- EP-A- 0 141 356
- DE-B- 1 059 436

## Beschreibung

Die vorliegende Erfindung betrifft ein Verfahren zum Trennen eines C₄-Kohlenwasserstoffgemisches enthaltend im wesentlichen 1,3-Butadien, Butene, Butane, und sonstige C₄-Kohlenwasserstoffe, in mindestens 4 Fraktionen, wobei
a) die Fraktion (a) im wesentlichen aus 1,3-Butadien,
b) die Fraktion (b) im wesentlichen aus Butenen
c) die Fraktion (c) im wesentlichen aus Butanen und
d) eine oder mehrere Fraktionen (d), die im wesentlichen aus den sonstigen C₄-Kohlenwasserstoffen bestehen,
durch Extraktivdestillation mittels N-Methyl-2-pyrrolidinon oder einer wässerigen Lösung von N-Methyl-2-pyrrolidinon (MMP),
wobei man
1. das gasförmige C₄-Kohlenwasserstoffgemisch zunächst mit NMP in einer Extraktionszone (I) in Kontakt bringt, wobei das Mengenverhältnis NMP zu C₄-Kohlenwasserstoffgemisch 5:1 bis 20:1 beträgt, das 1,3-Butadien und die sonstigen C₄-Kohlenwasserstoffe vom NMP im wesentlichen vollständig absorbiert werden, die Butene und Butane jedoch im wesentlichen in der Gasphase verbleiben;
2. die nicht-absorbierten Butene und Butane (Gasstrom bc) und die in Schritt 1 gebildete Extraktionslösung (Extraktionslösung ad) getrennt aus der Extraktionszone (I) entfernt;
3. die Extraktionslösung (ad) in eine Desorptionszone (I) mit gegenüber der Extraktionszone (I) vermindertem Druck und/oder erhöhter Temperatur überführt und dabei aus der Extraktionslösung (ad) 1,3-Butadien desorbiert, wobei der Hauptteil der sonstigen C₄-Kohlenwasserstoffe in der flüssigen Phase verbleibt;
4. die in Stufe 3 gebildete Extraktionslösung (Extraktionslösung d) und das desorbierte 1,3-Butadien (Fraktion a) getrennt aus der Desorptionszone (I) entfernt und gegebenenfalls einen Teil der Fraktion (a) in die Extraktionszone I zurückführt;
5. die Extraktionslösung (d) in eine zweite Desorptionszone (II) mit gegenüber der Desorptionszone (I) vermindertem Druck und/oder erhöhter Temperatur und mit einem Druck- und/oder Temperaturgradienten überführt und dabei aus der Extraktionslösung (d) noch darin verbliebenes 1,3-Butadien und die sonstigen C₄-Kohlenwasserstoffe mindestens als 2 getrennte Fraktionen (d) fraktioniert desorbiert, wobei in mindestens einer der Fraktionen (Fraktionen d) der Anteil der sonstigen C₄-Kohlenwasserstoffe mindestens 10fach höher ist als in der Extraktionslösung (d), bezogen auf den Anteil aller C₄-Kohlenwasserstoffe, und in mindestens einer der Fraktionen (Fraktionen dR) der Anteil der sonstigen C₄-Kohlenwasserstoffe entsprechend niedriger ist als in den Fraktionen (d), bezogen auf den Anteil aller C₄-Kohlenwasserstoffe,
6. das in der Desorptionszone (II) gebildete NMP, das im wesentlichen frei ist von C₄-Kohlenwasserstoffen, und die Fraktionen (d) und (dR) getrennt aus der Desorptionszone II entfernt und eine oder mehrere Fraktionen (dR) in die Desorptionszone (I) zurückführt,
7. den Gasstrom (bc) zunächst mit dem in Schritt 6 gebildeten NMP in einer Extraktionszone (II) in Kontakt bringt, wobei die Butene vom NMP im wesentlichen vollständig absorbiert werden, die Butane jedoch im wesentlichen in der Gasphase verbleiben;
8. die nicht-absorbierten Butane (Fraktion c) und die in Schritt 1 gebildete Extraktionslösung (Extraktionslösung b) aus der Extraktionszone (II) entfernt;
9. die Extraktionslösung (b) in eine Desorptionszone (III) mit gegenüber der Extraktionszone (II) vermindertem Druck und/oder erhöhter Temperatur überführt und dabei aus der Extraktionslösung (b) die Butene desorbiert;
10. das in Schritt 9 gebildete NMP, das im wesentlichen frei von C₄-Kohlenwasserstoffen ist, und die desorbierten Butene (Fraktion b) aus der Desorptionszone (III) entfernt;
11. das in Schritt 9 gebildete NMP in eine der Extraktionszonen zurückführt.
12. einen Teil der 1,3-Butadienfraktion (a), die man aus der Desorptionszone (I) entfernt, in die Extraktionszone (I) zurückführt (Fraktion a1) und den anderen Teil (Fraktion a2) nochmals in eine Extraktionszone (III) mit NMP in Kontakt bringt, das gemäß Schritt 6 des Anspruchs 1 zurückgewonnen wurde, wobei das Verhältnis an NMP zur Rohbutadienfraktion (a2) 1:3 bis 1:7 beträgt und ein Teil der Fraktion (a2) und der überwiegenden Teil an sonstigen C₄-Kohlenwasserstoffen, die noch als Verunreinigung in der Fraktion (a2) enthalten sind, vom NMP absorbiert werden (Extraktionslösung ax); und
13. den nicht absorbierten Teil der Fraktion (a2) (Fraktion a3) aus der Extraktionszone getrennt entfernt und die Extraktionslösung (ax) in die Extraktionszone (I) zurückführt.

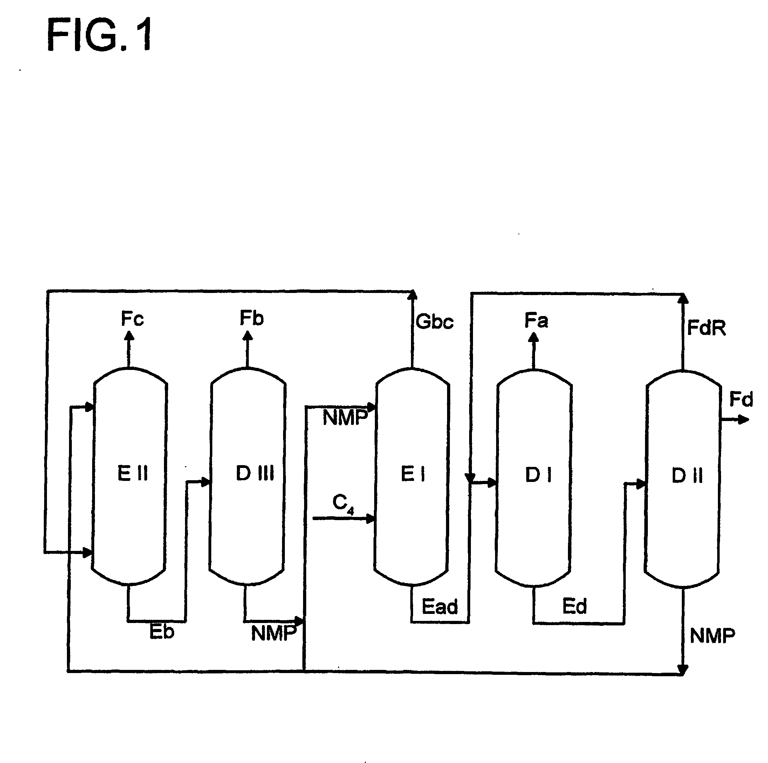

Dieses Verfahren ist in Fig. 1 schematisch dargestellt.

Ein Verfahren zur Abtrennung von 1,3-Butadien aus einem C₄-Kohlenwasserstoffgemisch ist beispielsweise aus der DE-A-2724365 bekannt. Bei diesem Verfahren wird kurz gesagt aus einem C₄-Kohlenwasserstoffgemisch, das Butane, Butene, 1,3-Butadien und sonstige C₄-Kohlenwasserstoffe enthält, durch Extraktivdestillation mit NMP als Absorptionsmittel über verschiedene Absorptions- und Desorptionsstufen eine Butane-Butene-Mischfraktion, eine 1,3-Butadienfraktion und eine Fraktion, die die übrigen Gemisch C₄-Kohlenwasserstoffe enthält, gewonnen. Das benötigte NMP durchläuft im Rahmen des Gesamtverfahrens einen geschlossenen Kreislauf. NMP, das keine C₄-Kohlenwasserstoffe mehr enthält (entladenes NMP), wird am Anfang eines Cyclus zunächst mit dem C₄-Kohlenwasserstoffgemisch beladen, durchläuft die verschiedenen Absorptions- und Desorptionsstufen, bis am Ende eines Cyclus entladenes NMP bereitgestellt wird, indem man die C₄-Kohlenwasserstoffe vollständig desorbiert. Das Verfahren zeichnet sich dadurch aus, daß die einzelnen Stufen über indirekte Wärmeaustauschprozesse besonders günstig verkoppelt sind.

Die Abtrennung von 1,3-Butadien und 2-Butenen in getrennten Fraktionen sowie die Abtrennung von 1,3-Butadien und Acetylenen in getrennten Fraktionen aus C₄-Kohlenwasserstoffgemischen ist in von V. A. Gorshkov et al in der Druckschrift "The Soviet Chemical Industry", No. 11, November 1971 beschrieben.

Die EP-A-141356 betrifft ebenfalls die Abtrennung von einer 1,3-Butadienfraktion, aus einem C₄-Kohlenwasserstoffgemisch mittels Extraktivdestillation unter Verwendung von NMP. Die Verwendung von Kolonnen, bei denen Absorptions- und Desorptionszone in jeweils einer einzigen Kolonne integriert sind, machen dieses Verfahren besonders wirtschaftlich.

Aus der EP-A-5788 ist ein Verfahren zur Abtrennung einer 1,3-Butadien- und einer Butinfraktion aus einem C₄-Kohlenwasserstoffgemisch mittels Extraktivdestillation unter Verwendung von NMP bekannt.

Die EP-A-9630 betrifft ein Verfahren zur getrennten Abtrennung von Styrol und 1,3-Butadien aus einer Mischung, die ansonsten C₄-Kohlenwasserstoffe enthält, wobei man zunächst das Styrol aus dem Gemisch durch Destillation abtrennt und 1,3-Butadien aus der verbleibenden Mischung mittels Extraktivdestillation abtrennt.

Aus der US 5242550 ist die Trennung einer Butene-Butane-Mischung mittels Extraktivdestillation mittels NMP als Absorptionsmittel bekannt.

In der EP-A-149145 ist ein Verfahren zur Trennung eines C₄-Kohlenstoffgemisches durch Extraktiv destillation beschrieben welches Verfahren beispielsweise zum Trennen eines 1,3-Butadien enthaltenden C₄ -Kohlenwasserstoffgemisches angewendet werden kann.

Aufgabe der vorliegenden Erfindung war es, ein Verfahren bereitzustellen, mit dem die Trennung eines C₄-Kohlenwasserstoffgemisches in eine Butane-, Butene-, 1,3-Butadien-Fraktion sowie eine Fraktion, die die sonstigen C₄-Kohlenwasserstoffe enthält, besonders effizient und wirtschaftlich möglich ist. Insbesondere sollen bei diesem Verfahren die benötigten Energiemengen und die Investitionskosten besonders gering sein.

Demgemäß wurde das eingangs beschriebene Verfahren gefunden.

Das Verfahren ist auf C₄-Kohlenwasserstoffgemische anwendbar, die 1,3-Butadien, Butene, Butane, und sonstige C₄-Kohlenwasserstoffe sowie geringfügige Mengen an Verunreinigungen an C₃- und C₅-Kohlenwasserstoffen enthalten.

Solche C₄-Kohlenwasserstoffgemische werden z.B. als C₄ Fraktionen bei der Herstellung von Ethylen und/oder Propylen durch thermisches Spalten einer Petroleumfraktion z.B. von verflüssigtem Petroleumgas (LPG), Leichtbenzin (Naphta), Gasöl oder dgl. als Kohlenwasserstoff-Fraktion erhalten. Weiterhin werden solche C₄-Fraktionen bei der katalytischen Dehydrierung von n-Butan und/oder n-Buten erhalten. die C₄ Fraktionen erhalten in der Regel Butane, n-Buten, Isobuten, 1,2 Butadien, Vinylacetylen, Ethylacetylen, 1,2-Butadien und gegebenenfalls geringe Mengen an C₅-Kohlenwasserstoffen, wobei der 1,3-Butadiengehalt im allgemeinen 10 bis 80 Gewichtsprozent, vorzugsweise 20 bis 70 Gewichtsprozent, insbesondere 30 bis 60 Gewichtsprozent beträgt, während der Gehalt der C₄-Fraktionen an Vinylacetylenen, Äthylacetylen und 1,2-Butadien (im folgenden Text "sonstige Kohlenwasserstoffe" genannt) insgesamt 5 Gewichtsprozent im allgemeinen nicht übersteigt.

Das erfindungsgemäße Verfahren läßt sich mit Vorteil besonders auf solche C₄-Kohlenwasserstoffgemische anwenden, die
- 10 bis 80 Gew.-% 1,3-Butadien;
- 10 bis 60 Gew.-% Butene;
- 5 bis 40 Gew.-% Butane;
- 0,1 bis 5 Gew.-% sonstige C₄-Kohlenwasserstoffe und
- 0 bis maximal 5 Gew.-% C₃- und C₅-Kohlenwasserstoffe enthalten.

Bei dem als selektiven Lösungsmittel eingesetzten N-Methyl-2-pyrrolidinon bzw. dessen wässeriger Lösung (N-Methyl-2-pyrrolidinon und dessen wässerige Lösung nachfolgend kurz als "NMP" abgekürzt) handelt es sich im allgemeinen um übliche technische Ware, die bis zu 15 Gew.-% Wasser enthalten kann.

Die Extraktionszonen werden bevorzugt in Form von Kolonnen ausgeführt, durch die man die Gasströme im Gegenstrom zum NMP leitet.

In Schritt 1 wird das zu trennende C₄-Kohlenwasserstoffgemisch zunächst gasförmig mit NMP in eine Extraktionszone (I) eingespeist und dort miteinander in Kontakt gebracht, wobei das 1,3-Butadien und die sonstigen C₄-Kohlenwasserstoffe vom NMP im wesentlichen vollständig absorbiert werden, die Butene und Butane jedoch im wesentlichen in der Gasphase verbleiben. Bei dem eingespeisten NMP und C₄-Kohlenwasserstoffgemisch beträgt das Mengenverhältnis NMP zu C₄-Kohlenwasserstoffgemisch 5 : 1 bis 20 : 1 in die Extraktionszone (I).

Für diesen Extraktionsschritt eignen sich die allgemein bekannten Extraktionsverfahren.

Aus der Extraktionszone (I) wird im allgemeinen ein Gasstrom, der vor allem nicht absorbierte Butane und Butene sowie, falls C₃- und C₅-Kohlenwasserstoffe als Verunreinigung im C₄-Gemisch enthalten sind, Propan, Propen und Propadien sowie Spuren an C₅-Kohlenwasserstoffen (Gasstrom bc), am Kopf der Kolonne und die Extraktionslösung (Extraktionslösung ad) aus dem Sumpf der Kolonne entfernt.

Die Extraktionslösung (ad) enthält im allgemeinen nur noch 0 bis 2 Gew.-% Butene und Butane, dazu, falls vorhanden, Propin und/oder fast die Gesamtmenge an C₅-Kohlenwasserstoffen.

Der Gasstrom (bc) enthält neben den Butenen und Butanen im allgemeinen nur noch 0 bis 1 Gew-% des ursprünglich im C₄-Kohlenwasserstoffgemisch vorhandenen 1,3-Butadiens und der sonstigen C₄-Kohlenwasserstoffe.

Die Extraktionszone (I) ist im allgemeinen in Form einer Waschkolonne ausgeführt, mit Böden, Füllkörpern oder strukturierten Pakkungen als Einbauten. Diese weisen vorteilhafterweise 40 bis 80 theoretische Böden auf. Der Kolonnendruck ist abhängig von der Temperatur des Kühlmediums (Brunnenwasser, Flußwasser, Meerwasser, Kältemittel wie Flüssigpropylen, Flüssigammoniak oder Sole). Er liegt zwischen 2 und 6 bar, vorzugsweise bei 4,5 bar. Das Temperaturprofil in der Extraktionszone wird durch die Temperatur des NMP bestimmt. Es ist vorteilhaft, durch partielle Kondensation der Fraktion (bc) das Temperaturprofil abzusenken, da sich mit niedrigerer Temperatur die Trennleistung verbessert. Ein typischer Wert für die Kondensation liegt bei 20 %. Daraus resultiert eine Temperatur von 40 bis 60 C am Kopf der Kolonne.

Zur Desorption des 1,3-Butadiens aus der Extraktionslösung (ad) wird sie in eine Desorptionszone (I) mit gegenüber der Extraktionszone (I) vermindertem Druck und/oder erhöhter Temperatur überführt und dabei aus der Extraktionslösung (ad) 1,3-Butadien (1,3-Butadienfraktion a) desorbiert, wobei der Hauptteil der sonstigen C₄-Kohlenwasserstoffe, Propin und C₅-Kohlenwasserstoffe in der flüssigen Phase verbleibt.

Bevorzugt liegt in der Desorptionszone (I) der Druck auf gleicher Höhe wie in der Extraktionszone (I) und die Temperatur 20 bis 25°C höher als in der Extraktionszone (I).

Die aus der Desorptionszone (I) entfernte 1,3-Butadienfraktion (a) weist üblicherweise ein Reinheit von 95 bis 99 Gew.-% auf.

Die durch Desorption von 1,3-Butadien in der Desorptionszone (I) gebildete Extraktionslösung (d) wird anschließend aus der Desorptionszone (I) entfernt und in eine zweite Desorptionszone (II) mit gegenüber der Desorptionszone (I) vermindertem Druck und/oder erhöhter Temperatur überführt. Während der Überführung der Extraktionslösung (d) von Desorptionszone (I) nach (II) wird vorteilhafterweise eine Wärmetauscherzone durchlaufen, bei der ein Teil der Kohlenwasserstoffe in der Extraktionslösung (d) verdampft, wobei dieser Gasstrom unmittelbar wieder in den Sumpf der Desorptionszone (I) eingespeist wird. Druck und Temperatur werden dabei so gewählt, daß praktisch alle noch im NMP verbliebenen C₄-Kohlenwasserstoffe desorbiert werden, sie liegen im allgemeinen bei 1,5 bar und 150°C.

In der Desorptionszone II wird aus der Extraktionslösung (d) noch darin verbliebenes 1,3-Butadien und die sonstigen C4-Kohlenwasserstoffe sowie gegebenenfalls darin noch vorhandene und C₅-Kohlenwasserstoffe mindestens als 2 getrennte Fraktionen (d) fraktioniert desorbiert, wobei in mindestens einer der Fraktionen (Fraktion d) der Anteil der sonstigen C₄-Kohlenwasserstoffe mindestens 10fach, im allgemeinen 10 bis 100fach, bevorzugt 20 bis 80fach höher ist als in der Extraktionslösung (d), bezogen auf den Anteil aller C₄-Kohlenwasserstoffe, und in mindestens einer der Fraktionen (Fraktionen dR) der Anteil der sonstigen C₄-Kohlenwasserstoffe niedriger ist als in den Fraktionen (d), bezogen auf den Anteil aller C₄-Kohlenwasserstoffe. Bevorzugt wird in der Desorptionszone (II) die Kohlenwasserstoffe der Extraktionslösung (d) in je eine Fraktion (d) und eine Fraktion (dR) aufgetrennt, wobei die Fraktion (d) bevorzugt im wesentlichen zu mindestens aus 20 Gew.-%, besonders bevorzugt zu 20 bis 40 Gew.-% aus sonstigen C₄-Kohlenwasserstoffen und ansonsten aus Butadien besteht und die Fraktion (dR) im wesentlichen aus mehr als 80 Gew.-%, besonders bevorzugt zu 85 bis 95 Gew.-% Butadien und ansonsten aus sonstigen C₄-Kohlenwasserstoffen.

Das in der Desorptionszone (II) gebildete NMP, das im wesentlichen frei ist von Kohlenwasserstoffen, und die Fraktionen (d) und (dR) werden getrennt aus der Desorptionszone II entfernt und eine oder mehrere der Fraktionen (dR) in die Desorptionszone (I) zurückführt, z.B. in den Sumpf der Waschkolonne.

Bevorzugt wird das Druckgefälle hierbei über einen Verdichter überwunden. Die Fraktion (d) wird üblicherweise mit Wasser (Kondensat) im Gegenstrom behandelt, um den Großteil des in ihm enthaltenen NMP zu absorbieren.

Im allgemeinen beträgt das Massenverhältnis der in die Desorptionszone (I) zurückgeführten Fraktionen zu den dem System entnommenen 20 : 1 bis 80 : 1.

Die Desorptionszone (II) besteht im allgemeinen aus einer Hauptkolonne mit Seitenkolonne. Beide sind als Waschkolonnen konzipiert. Die Hauptkolonne enthält im allgemeinen Füllkörperpackungen, da sich hier deren geringer Druckverlust besonders günstig auswirkt. Die Hauptkolonne sollte 10 bis 15 theoretische Böden aufweisen. Die Seitenkolonne weist im allgemeinen 10 praktische Böden auf. Der Druck liegt im allgemeinen bei 1,5 bis 1,6 bar; die Temperatur am Sumpf der Hauptkolonne beträgt 140 bis 150 C und an deren Kopf 80 bis 100°C. Während die Fraktionen (d) als Seitenstrom bevorzugt bei 130 bis 140°C entnommen werden, werden die Fraktionen (dR) üblicherweise über Kopf abgezogen.

Wird eine 1,3-Butadienfraktion mit einer besonders hohen Reinheit gewünscht, geht man bevorzugt auf folgende Weise vor:
- Die 1,3-Butadienfraktion (a), die man aus der Desorptionszone (I) entfernt, wird in 2 Teilströme Fraktion (a1) und (a2) aufgespalten, wobei die Fraktion (a1) in die Extraktionszone I zurückgeführt wird (bevorzugt in den Sumpf der Extraktionskolonne I geleitet wird) und die Fraktion (a2) nochmals in einer Extraktionszone (III) mit NMP in Kontakt gebracht, das aus der Desorptionszone (II) oder (III) zurückgewonnen wurde, wobei ein Teil der Fraktion (a2) und der überwiegende Teil an sonstigen C₄-Kohlenwasserstoffen, die noch als Verunreinigung in der Fraktion (a2) enthalten sind, vom NMP absorbiert werden (Extraktionslösung ax).
- Der nicht absorbierte Teil der Fraktion (a2) (Fraktion a3) wird aus der Extraktionszone getrennt entfernt und die Extraktionslösung (ax) in die Extraktionszone (I) zurückfuhrt.

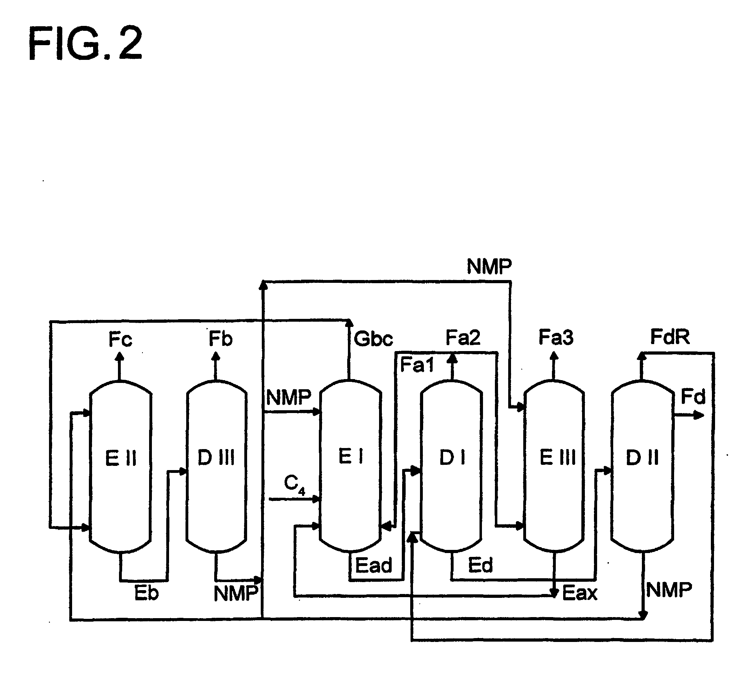

Diese Variante ist in Fig. 2 verdeutlicht.

Das Massenverhältnis an NMP zu 1,3-Butadienfraktion (a) entspricht im allgemeinen 1 : 3 bis 1 : 7, abhängig von der Zusammensetzung des C₄-Einsatzgemisches und der Spezifikationen der Fraktion (a3).

Das Verhältnis der Stoffströme der Fraktionen (a1) und (a2) liegt üblicherweise bei 1:1 bis 4:1.

Die 1,3-Butadienfraktion (a3) enthält als Verunreinigungen vor allem noch C₃-und C₅-Kohlenwasserstoffe sowie 1,2-Butadien. Diese Verunreinigungen werden im allgemeinen anschließend in zwei konventionellen Destillationskolonnen abgetrennt.

Für die Extraktionszone (III) gilt sinngemäß das gleiche wie für die Extraktionszone (I), was die Ausführung der Extraktionskolonne und die Parameter Druck und Temperatur betrifft. Das Verhältnis an eingespeistem MMP zur Rohbutadienfraktion (a2) entspricht 1 : 3 bis 1 : 7.

Das nicht-absorbierte 1,3-Butadien und aus der Extraktionszone (III) abgezogene 1,3-Butadien weist üblicherweise eine Reinheit von mehr als 98 Gew.-% auf.

Der Gasstrom (bc), optional unter Hinzufügen des externen Zusatzstromes (Gasstrom Zbc, wird zunächst mit dem in der Desorptionszone (II) rückgewonnenen NMP in einer Extraktionszone (II) in Kontakt gebracht, wobei die Butene vom NMP im wesentlichen vollständig absorbiert werden, die Butane jedoch im wesentlichen in der Gasphase verbleiben.

Die Extraktionszone (II) ist im allgemeinen in Form einer Waschkolonne ausgeführt, mit Böden, Füllkörpern oder strukturierten Packungen als Einbauten. Diese müssen 30 bis 70 theoretische Böden aufweisen, um eine hinreichend gute Trennwirkung zu erzielen. Der Druck in der Extraktionszone (II) wird so gewählt, daß der Gasstrom (bc) aus der Extraktionszone (I) ohne weitere technische Hilfsmittel in die Extraktionszone (II) gelangen kann.

Außerdem ist er abhängig vom zur Verfügung stehenden Kühlmedium, um die Fraktion (c) zu kondensieren. Ein typischer Wert für den Druck liegt bei 4,0 bar, Kühlung mit Wasser vorausgesetzt.

Mit Vorteil ist die Waschkolonne im Kolonnenkopf mit einer Rückwaschzone ausgerüstet, die beispielsweise 4 theoretische Böden umfaßt. Diese Rückwaschzone dient zur Rückgewinnung des in der Gasphase enthaltenen NMP mittels flüssigen Kohlenwasserstoffrücklaufes, wozu die Fraktion (c) zuvor kondensiert wurde. Gleichzeitig kann damit das Temperaturprofil in der Extraktionszone (III) beeinflußt werden. Wie bereits in Extraktionszone (I) erwähnt, gilt auch hier, daß eine niedrigere Temperatur die Trennleistung fördert. Typische Temperaturen am Kopf der Kolonne liegen zwischen 35 und 45°C.

Das Massenverhältnis NMP zu Gasstrom (bc), inclusiv optional Gasstrom Zbc, im Zulauf zur Extraktionszone (II) beträgt 10 : 1 bis 20 : 1, abhängig von den Spezifikationen der Fraktionen (b) und (c) und der Zusammensetzung des C₄-Einsatzgemisches sowie des Zusatzstromes Zbc.

In der Extraktionszone (II) wird eine gasförmige Butanfraktion (Fraktion c) und eine die Butenfraktion (Fraktion b) enthaltene Extraktionslösung (b) gebildet. Wird die Extraktivdestillation wie vorstehend beschrieben durchgeführt, so erhält man eine Fraktion (b), die bis zu 5 Gew.-% mit Butanen und eine Fraktion (c), die bis zu 15 Gew.-% mit Butenen verunreinigt ist.

Die Extraktionslösung (b) wird in eine Desprptionszone (III) mit gegenüber der Extraktionszone (II) vermindertem Druck und/oder erhöhter Temperatur überführt, wobei aus der Extraktionslösung (b) die Butene desorbiert werden. Die Desorption der Butene und der gegebenenfalls als Verunreinigung darin enthaltenen anderen C₄-Kohlenwasserstoffe kann grundsätzlich analog zu der Desorption der sonstigen C₄-Kohlenwasserstoffe in der Desorptionszone (II) erfolgen.

Die Desorptionszone (III) kann beispielsweise in Form einer Waschkolonne ausgeführt sein, die 5 bis 15, bevorzugt 8 bis 10 theoretische Stufen und eine Rückwaschzone mit beispielsweise 4 theoretischen Stufen aufweist. Diese Rückwaschzone dient zur Rückgewinnung des in der Gasphase enthaltenen NMP mittels flüssigen Kohlenwasserstoffrücklaufes, wozu die Fraktion (b) zuvor kondensiert wird. Vorteilhaft sind Füllkörperschüttungen als Einbauten vorzusehen. Der Druck am Kolonnenkopf liegt im allgemeinen bei 1,5 und 1,6 bar. Die Temperatur im Kolonnensumpf liegt im allgemeinen bei 130 bis 150°C.

Das in der Desorptionszone (III) rückgewonnene NMP wird in die Extraktionszonen (I), (II) und/oder (III) zurückgeführt.

Einen zusätzlicher Vorteil ergibt sich, wenn man das in der Desorptionszone (III) rückgewonnene NMP nur in die Extraktionszonen (I) und (II) und das in der Desorptionszone (II) rückgewonnene NMP im wesentlichen in die Extraktionszone (III) zurückleitet.

Der Vorteil hängt damit zusammen, daß die Abtrennung von Butenen/Butanen aus einem C₄-Kohlenwasserstoffgemisch mittels NMP leichter vonstatten geht als die Trennung eines Butene-Butane-Gemisches in zwei hoch reine Butene- und Butanefraktionen. Dabei bleibt es bei einem einzigen Lösungsmittelkreislauf.

Im Gegensatz zur Extraktionszone (III), die hoch reines ausgegastes NMP benötigt, braucht die Qualität des NMP für die Extraktionszonen (I) und (II) nicht so hoch zu sein. Das bedeutet wirtschaftlich einen Gewinn in der Hinsicht, daß der Ausgasungsgrad des NMP und damit der Verbrauch an externem Dampf zur Desorption von Kohlenwasserstoffen in der Desorptionszone (III) nicht so hoch zu sein braucht. Im Gegensatz zum NMP aus der Desorptionszone (II), wo 0 bis 10 Gew.-ppm an C₄-Kohlenwasserstoffen angestrebt werden, kann das NMP aus der Desorptionszone (III) durchaus 1000 und mehr Gew.-ppm aufweisen. Das stört die Produktreinheit der Fraktionen (b) und (c) nicht. Andererseits senkt aber ein Gehalt an Kohlenwasserstoffen die Siedetemperatur des LM. Da der Wärmeinhalt des NMP aus der Desorptionszone (III) aus wirtschaftlichen Gründen aber genutzt wird, kann das Absenken der Siedetemperatur durch Erhöhung des Restgehaltes an Kohlenwasserstoffen nicht beliebig weit vorangetrieben werden. Aus diesen Gründen resultieren die zuvor angegebenen Sumpftemperaturen von 130 bis 150 C. Bei einer Sumpf temperatur von 138 C resultiert ein Restgehalt an Kohlenwasserstoffen von ca. 800 Gew.-ppm.

Das erfindungsgemäße Verfahren läßt sich besonders wirtschaftlich ausüben, wenn man die Wärme des NMP, das man durch Aufkochen der Extraktionslösungen (b) und (d) gewinnt durch indirekten Wärmeaustausch in einer Wärmeaustauschzone, der Desorptionszone (I) (II) und/oder (III) zuführt und die Desorption in diesen Desorptionszonen dadurch bewirkt, daß man die Temperatur in der Desorptionszone (I) gegenüber der in der Extraktionszone (I), die Temperatur in der Desorptionszone (II) gegenüber der in der Desorotionszone (I) bzw. die Temperatur in der Desorptionszone (III) gegenüber der in der Extraktionszone (II) erhöht.

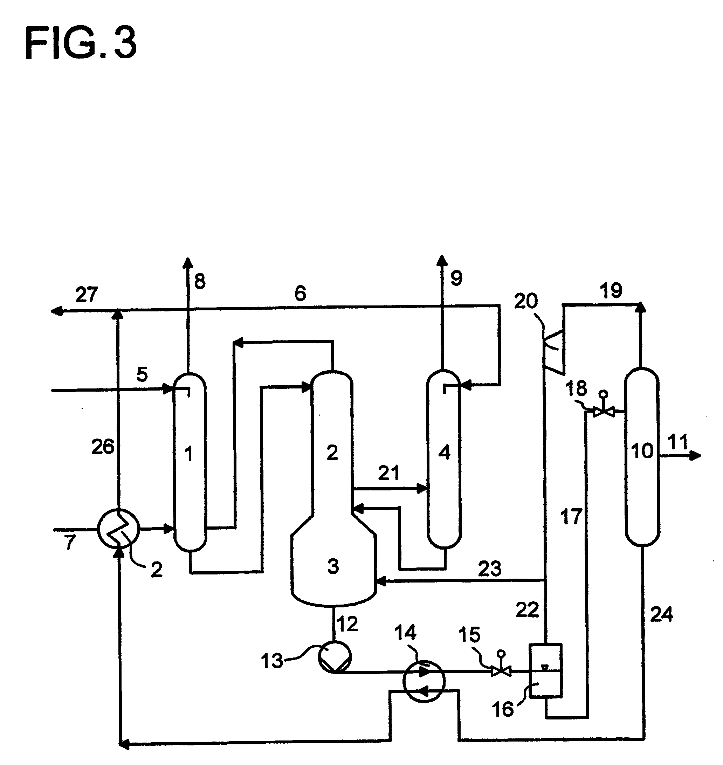

Die Abtrennung der Fraktion (a) (Butadien) aus dem C₄-Kohlenwasserstoffgemisch wird bevorzugt so ausgeführt, wie es in der DE-A-2724365 beschrieben ist. Besonders bevorzugt wird dieser Teil des Verfahrens so durchgeführt, wie es in Fig. 3 beschrieben ist.

Nach diese Vefahrensvariante wird folgendermaßen vorgegangen:

Die extraktive Destillation wird in mehr als einer Kolonne, im allgemeinen in zwei Kolonnen ausgeführt, die zusammen über mehr als 100 praktische Böden verfügen. Zweckmäßig wird man bei der Verwendung von zwei Kolonnen die oberhalb des Punktes, an dem das C₄-Kohlenwasserstoffgemisch in die extraktive Destillationszone eingeführt wird, liegende Absorptionsstufe in die erste Kolonne und die unterhalb des Einführungspunktes des Kohlenwasserstoffgemisches liegende Anreicherungsstufe in die zweite Kolonne verlegen, d.h. der Einführungspunkt für das Kohlenwasserstoffgemisch ist am Kopf der zweiten Kolonne oder vorzugsweise am Sumpf der ersten Kolonne. Vorzugsweise wird man zwischen Absorptionsstufe und Anreicherungsstufe keine Kompressionsstufe zwischenschalten, sondern solche Druckverhältnisse innerhalb der extraktiven Destillationszone aufrecht erhalten, wie sie sich von selbst in der extraktiven Destillationszone bei Fehlen von Kompressionsund/oder Druckminderungsstufen innerhalb der extraktiven Destillationszone einstellen, so daß der Druck am Boden der extraktiven Destillationszone entsprechend dem üblichen Druckverlust in den Kolonnen mindestens dem Druck am Kopf der extraktiven Destillationszone entspricht. In der Regel beträgt die Druckdifferenz zwischen Kopf und Boden der extraktiven Destillationszone 0,1 bis 3, vorzugsweise 0,2 bis 2 bar.

Im allgemeinen wird man in der extraktiven Destillationszone Drucke zwischen 1 bis 9, vorzugsweise 2 bis 8 bar, insbesondere 3 bis 7 bar anwenden. Die Drucke im unteren Drittel der extraktiven Destillationszone, d.h. dem Bereich, der von den unteren Böden der extraktiven Destillationszone eingenommen wird, die etwa einem Drittel der Gesamtbodenzahl der extraktiven Destillationszone entsprechen, betragen in der Regel 1,5 bis 9, vorzugsweise 2,5 bis 8, insbesondere 3,5 bis 7 bar.

Der aus den Extraktivdestillationszonen abgezogene Extrakt wird zunächst auf einen höheren Druck gebracht als der Druck in der Extraktivdestillationszone.

Dies kann beispielsweise mit einer Flüssigkeitspumpe erfolgen. Im allgemeinen erfolgt diese Druckerböhung im wesentlichen isotherm, d.h. es ergeben sich nur solche Temperaturänderungen, z.B. eine Temperaturerhöhung bis zu 1°C, die durch die zur Druckerhöhung führende Maßnahme, z.B. den Pumpvorgang, bedingt sind. Im allgemeinen wird man den Extrakt auf Drucke bringen, die 1 bis 20, vorzugsweise 2 bis 18, insbesondere 3 bis 15 bar über dem Druck in der extraktiven Destillationszone, insbesondere über dem Druck im unteren Drittel der extraktiven Destillationszone liegen.

Der unter erhöhtem Druck stehende Extrakt wird anschließend in einer Wärmeaustauschzone im indirekten Wärmeaustausch mit dem aus der Lösungsmittelwiedergewinnungszone als Sumpfprodukt erhaltenen selektiven Lösungsmittel erwärmt. Das selektive Lösungsmittel wird nach dem Wärmeaustausch in die extraktive Destillationszone zurückgeführt. Durch den Wärmeaustausch mit dem selektiven Lösungsmittel wird die Temperatur des Extraktes im allgemeinen um 5 bis 80, vorzugsweise 10 bis 70, insbesondere 15 bis 60°C erhöht.

Der erwärmte Extrakt wird danach durch Entspannungsverdampfung (Flashverdampfung) auf einen Druck entspannt, der mindestens dem Druck in der extraktiven Destillationszone, vorzugsweise mindestens dem Druck im unteren Drittel der extraktiven Destillationszone entspricht und höher liegt als der Druck in der nachgeschalteten Lösungsmittelwiedergewinnungszone. Maßgeblich für die Druckminderung ist, daß sich der bei der Entspannungsverdampfung bildende dampfförmige Anteil des Extraktes ohne Kompressionsstufe in die extraktive Destillationszone zurückführen läßt. Dementsprechend wird in der Entspannungsverdampfung in der Regel auf Drucke entspannt, die 0,05 bis 2,0, vorzugsweise 0,1 bis 1 bar über dem Druck an der Einführungsstelle des dampfförmigen Anteils des Extraktes in die extraktive Destillationszone liegen. Die Entspannungsverdampfung wird z. B. in einer Vorrichtung aus Druckminderungsventil auf einem adiabatischen Verdampfer durchgeführt, wobei gegebenenfalls ein Phasenabscheidegefäß zur besseren Trennung der sich bei der Entspannungsverdampfung ausbildenden dampfförmigen und flüssigen Phase nachgeschaltet wird.

Die Kombination von Wärmeaustauschzone für den Wärmeaustausch zwischen dem Extrakt aus der extraktiven Destillationszone und dem aus der Lösungsmittelwiedergewinnungszone rückgeführten selektiven Lösungsmittel mit der nachgeschalteten Entspannungsverdampfung kann in einer Stufe angewendet werden. Es ist jedoch auch möglich, mehr als eine, z.B. 2 bis 4, vorzugsweise 2 bis 3 solcher Kombinationen, zweckmäßig in Hintereinanderschaltung anzuwenden. Durch Anwendung von mehr als einer dieser Wärmeaustausch-Entspannungverdampfungsstufen und Rückführung der so erhaltenen Teilströme zu unterschiedlichen Zulaufstellen der extraktiven Destillationszone lassen sich die erforderliche Trennleistung der extraktiven Destillation und die Abmessungen der extraktiven Destillationskolonne reduzieren. Es ist weiter möglich, zwischen der letzten Entspannungsverdampfungszone und der Lösungsmittelwiedergewinnungszone eine weitere Wärmeaustauschzone zwischenzuschalten.

Der sich in der bzw. den Entspannungsverdampfungszonen bildende dampfförmige Anteil des Extraktes, der im allgemeinen 20 bis 80 vorzugsweise 40 bis 70 Gew.-%, der Kohlenwasserstoffe im Extrakt beträgt, wird in die Extraktivdestillationszone zurückgeführt. Im allgemeinen wird die rückgeführte dampfförmige Phase in das untere Drittel der extraktiven Destillationszone eingeleitet, vorzugsweise am Sumpf der extraktiven Destillationszone, z.B. an einem Punkt, der sich in etwa in Höhe des untersten Kolonnenbodens befindet. Bei der stufenweisen Entspannungsverdampfung können in den einzelnen Stufen enthaltenen dampfförmigen Anteile getrennt oder nach ihrer Vereinigung in die Extraktivdestillationszone zurückgeführt werden.

Die nach der Entspannungsverdampfung verbleibende flüssige Phase des Extraktes aus der extraktiven Destillationszone wird einer Lösungsmittelwiedergewinnungszone zugeführt, die bei niedrigerem Druck als der Druck in der Entspannungsverdampfungszone betrieben wird. Die Entspannung der verbleibenden flüssigen Extraktphase auf den niedrigeren Druck in der Lösungsmittelwiedergewinnungszone erfolgt zweckmäßig durch ein zwischengeschaltetes Druckminderungsventil. Im allgemeinen ist der Druck in der Lösungsmittelwiedergewinnungszone 0,1 bis 8, vorzugsweise 0,5 bis 7, insbesondere 1 bis 6 bar niedriger als der Druck in der bzw. den Entspannungsverdampfungszonen. Die Lösungsmittelwiedergewinnungszone kann z.B. als Ausgaser oder als Lösungsmittelabstreifer betrieben werden. Der Lösungsmittelwiedergewinnungszone wird im allgemeinen Wärme zugeführt, beispielsweise über einen indirekten Wärmetauscher mit Wasserdampf (Aufkocher).

Das als Sumpfprodukt der Lösungsmittelwiedergewinnungszone erhaltene, von den Kohlenwasserstoffen befreite NMP wird über die Wärmeaustauschzone, in der der Wärmetausch mit dem Extrakt aus der extraktiven Destillationszone erfolgt, in die Extraktionsstufen (I) und (III) zurückgeführt.

Das aus der Lösungsmittelwiedergewinnungszone erhaltene, die Kohlenwasserstoffe enthaltene Produkt, welches im allgemeinen als Kopfstrom oder als Kopf- und Seitenstrom abgezogen wird, durchläuft teilweise oder ggf. ganz zunächst eine Kompressionsstufe und wird nach der Kompression der Extraktivdestillationszone zugeführt. In der Kompressionszone wird der Kohlenwasserstoffstrom auf einen Druck verdichtet, der mindestens dem Druck in der Extraktivdestillationszone entspricht. Im allgemeinen wird der Kohlenwasserstoffstrom auf Drucke verdichtet, die 0,05 bis 2, vorzugsweise 0,1 bis 1 bar über dem Druck an der Einführungsstelle des dampfförmigen Anteils des Extraktes in die extraktive Destillationszone liegen.

Fig 3 ist ein schematisches Diagramm einer Ausführungsform der bevorzugten Variante. Bei dieser Ausführungsform sind 2 Extraktivdestillationszonen hintereinandergeschaltet. Die erste Extraktivdestillationszone wird von Kolonne 1 sowie dem oberen verjüngten Kolonnenteil 2 gebildet, während die zweite Extraktivdestillationszone von der Kolonne 4 und dem unteren Kolonnenteil 3 gebildet wird. Das NMP wird durch Leitung 5 im oberen Teil von Kolonne 1 zugegegeben und durch Leitung 6 im oberen Teil der Kolonne 4 zugegeben. Durch Leitung 7 wird ein C₄-Kohlenwasserstoffgemisch am Sumpf der Kolonne 1 zugeleitet.

Außerdem stehen die Kolonnen 2/3 und 4 in direkter Verbindung. Ein gasförmiger Teilstrom wird der Kolonne 2 entnommen und im Gegenstrom mit LM über Leitung 6 gewaschen.

Am Kopf der Kolonne 1 wird durch Leitung 8 ein Raffinat, das im wesentlichen aus Butenen und Butanen besteht, abgezogen.

Am Kopf der Kolonne 4 wird durch Leitung 9 ein im wesentlichen reines 1,3-Butadien abgezogen.

Über den Seitenabzug der Kolonne 10 wird durch Leitung 11 ein im wesentlichen die sonstigen Kohlenwasserstoffe und sonstige Verunreinigungen enthaltener Gasstrom entfernt.

Der Druck im Kolonnenteil 3 beträgt ca. 5 bar. Der über Leitung 12 abgezogene Extrakt wird durch Flüssigkeitspumpe 13 auf einen Druck von 15 bar gebracht und anschließend im Wärmetauscher 14 von 70?C auf 125?C mit dem aus Ausgaser 10 über Leitung 24 abgezogenen, im wesentlichen von C₄-Kohlenwasserstoffen freien NMP erwärmt. Der erwärmte Extrakt wird anschließend durch Druckminderventil 15 geleitet und auf einen Druck etwas über 5 bar entspannt. Während die im Phasenabscheidebehälter 16 gebildete gasförmige Phase sofort wieder über Leitung 22 und 23 in die Kolonne 3 zurückgeführt wird, wird die nach der Entspannungsverdampfung erhaltene flüssige Phase des Extraktes über Leitung 17 einem weiteren Druckminderungsventil 18 zugeführt, wo ein Druckabfall auf das Druckniveau der Kolonne 10 erfolgt, üblicherweise 1,5 bar.

Am Kopf von Kolonne 10 wird über Leitung 19 ein Kohlenwasserstoffstrom abgezogen, der nach Verdichtung im Kompressor 20 über Leitung 23 auch dem Sumpf der Kolonne 3 zugeführt wird. Wichtig ist aus sicherheitstechnischen Gründen dabei, daß der Gasstrom 19 vor Eintritt in den Kompressor 20 durch Wärmetausch (in Fig. 3 nicht dargestellt) so abgekühlt wird, daß die Temperatur des Gasstromes nach Austritt aus dem Kompressor 110°C nicht überschreitet. Üblicherweise kühlt man den Gasstrom auf 45°C ab.

Das über Leitung 24 abgezogene und im Wärmetauscher 14 abgekühlte, von C₄-Kohlenwasserstoffen nahezu freie NMP wird über Leitung 25 dem Wärmetauscher 2 zugeführt. Danach passiert es einen weiteren Wärmetauscher (in Fig. 3 nicht dargestellt), in dem die Temperatur des LM auf 38°C eingestellt wird. Anschließend wird die Lösungsmittelmenge, wie sie über Leitung 26 ankommt, in zwei Teilströme aufgeteilt: Leitung 6 führt zur Kolonne 4, während Leitung 27 in der zusätzlichen Extraktivdestillationszone (III) der Butene-Butane-Trennung endet. Von dort kommt das LM über Leitung 5 wieder zurück.

Aus Gründen der zeichnerischen Vereinfachung sind auch alle zuvor erwähnten Rückwaschzonen mit den Rückläufen an Flüssigkohlenwasserstoffen auf die Kolonnen 1 und 4 fortgelassen. Auch fehlt in der Fig. 3 die zuvor erwähnte Seitenkolonne an der Hauptkolonne 10.

### Beispiel

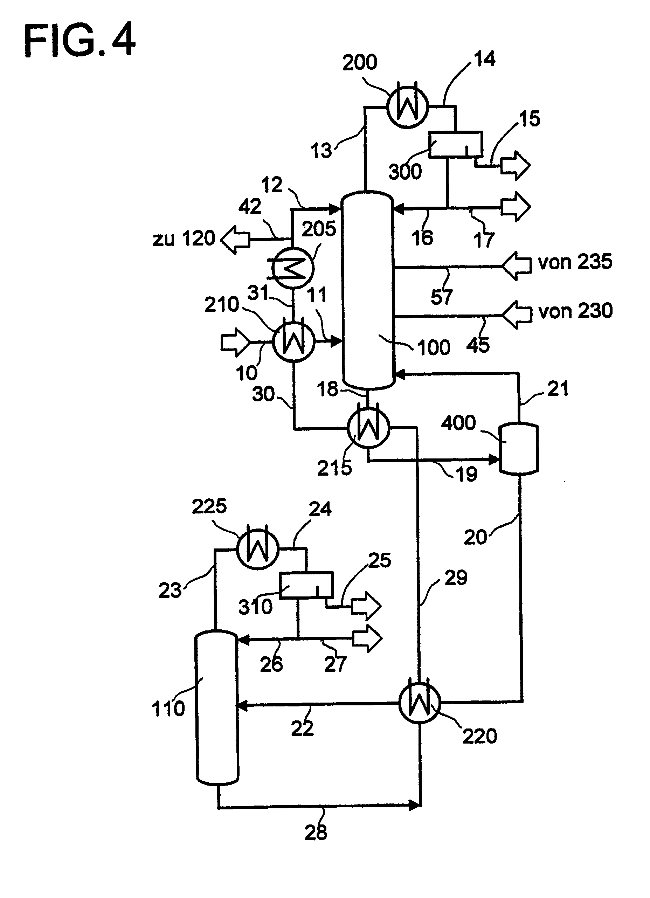

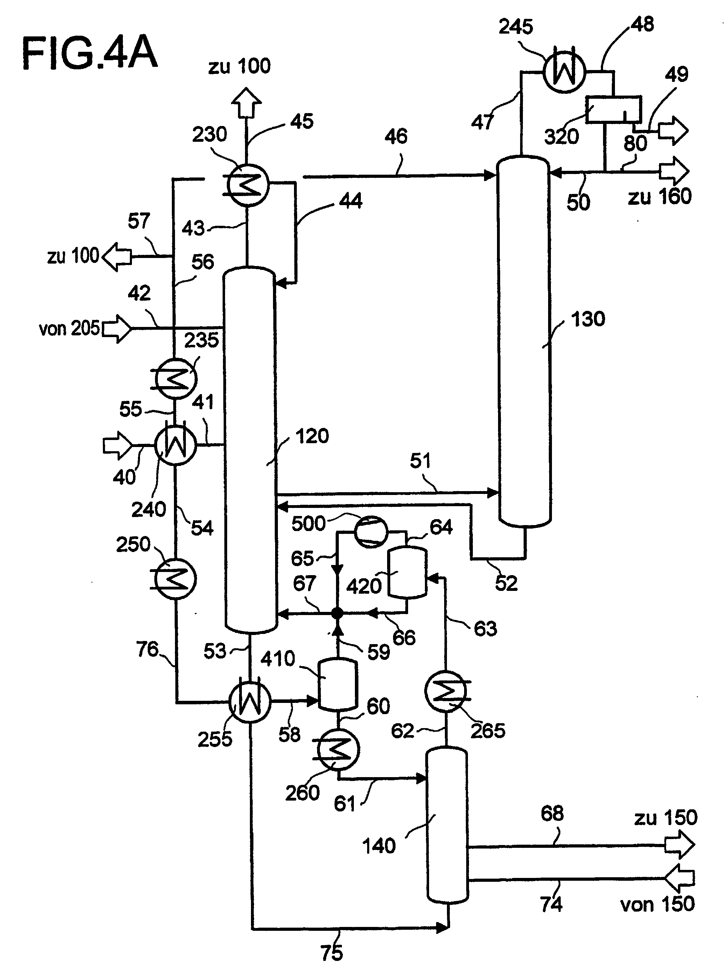

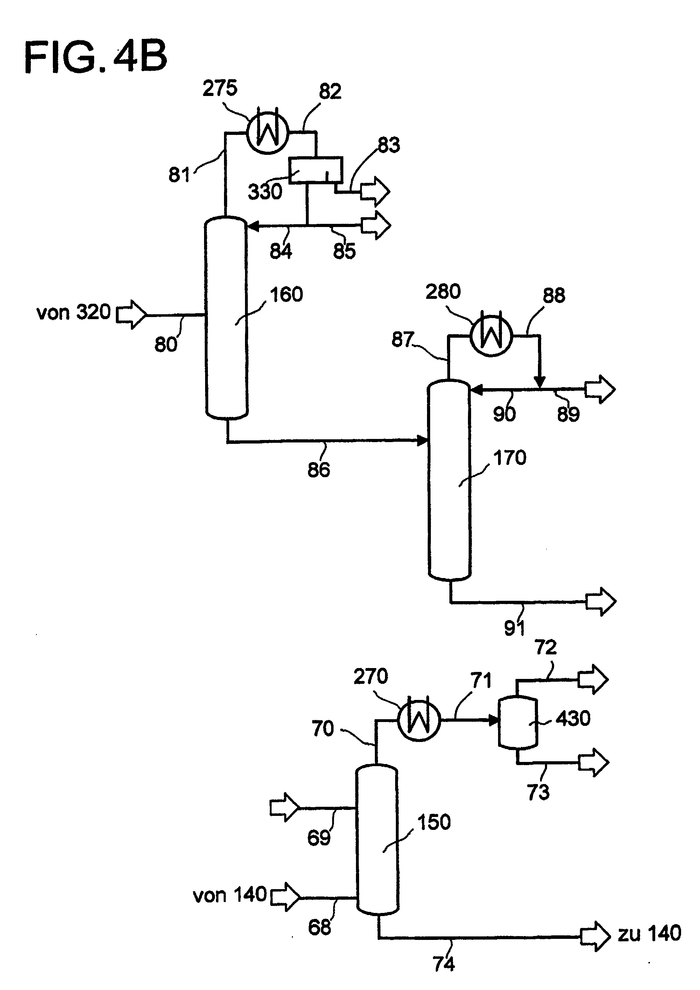

Gegenüber Fig. 3 ist das Verfahrensschema des Beispiels wesentlich umfangreicher (Fig. 4), selbst wenn auch hier alle Pumpen bildlich weggelassen sind. Dafür sind neben den Kolonnen alle Wärmetauscher aufgeführt, da sie maßgeblich an der Wirtschaftlichkeit des Verfahrens beteiligt sind. Desgleichen sind alle Phasenabscheider ins Schema mit aufgenommen. Die Systematik der Nummernzuordnung kann gleichfalls Fig. 4 entnommen werden.

Die Extraktionszone (I) sowie die Desorptionszone (I) verbergen sich hinter der Kolonne 120. Extraktionszone (III) ist Kolonne 130. Die Desorptionszone (II) wird durch die beiden Kolonnen 140 und 150 dargestellt. Extraktionszone (II) ist die Kolonne 100 und Desorptionszone (III) Kolonne 110. Zusätzlich, weil zum Gesamtverfahren gehörend, wurden die beiden Schlußdestillationskolonnen 160 und 170 berücksichtigt, in denen das Rohbutadien endgültig auf Spezifikation gebracht wird.

Das Verfahren besitzt 3 Eingangs ströme:
- Strom 10:: Zusatzstrom Zbc, Butene und Butane enthaltend
- Strom 40:: C₄-Einsatz
- Strom 69:: Kondensatzugabe zwecks Reduktion des NMP-Verlustes

Zusammensetzung [Gew.-%] und Mengenangabe [kg/h] von Strom 10:

| | |
|---|---|
| n-Butan | 26,0 |
| i-Butan | 9,5 |
| n-Buten | 42,0 |
| trans-Buten-2 | 13,0 |
| cis-Buten-2 | 9,5 |

Menge = 4200.

Zusammensetzung [Gew.-%] und Mengenangabe [kg/h] von Strom 40:

| | |
|---|---|
| Propan | 0,1 |
| Propen | 0,1 |
| Propadien | 0,05 |
| Propin | 0,15 |
| n-Butan | 7,3 |
| i-Butan | 4,0 |
| n-Buten | 14,0 |
| i-Buten | 24,6 |
| trans-Buten-2 | 4,5 |
| cis-Buten-2 | 3,5 |
| 1,3-Butadien | 40,0 |
| 1,2-Butadien | 0,45 |
| Ethylacetylen | 0,2 |
| Vinylacetylen | 0,75 |
| i-Pentan | 0,1 |
| 3-Methylbuten-2 | 0,1 |
| 2-Methylbuten-2 | 0,1 |

Menge = 15000.
Mengenangabe für Strom 69 [kg/h] :
Menge = 1100.

Lösungsmittelbedarf für die 3 Extraktionszonen [kg/h] und deren Zusammensetzung [Gew.-%] : (KW = Kohlenwasserstoffe)

| | | |
|---|---|---|
| (IExtr.-Zone )Strom 42 = 165000. mit | NMP | 91,63 |
| | Wasser | 8,29 |
| | Summe KW | 0,08 |
| Extr.-Zone(III)Strom 46 = 35000. mit | NMP | 91,7 |
| | Wasser | 8,3 |
| | Summe KW | 1 Gew.-ppm |
| Extr.-Zone (II) Strom 12 = 100000. mit | NMP | 91,63 |
| | Wasser | 8,29 |
| | Summe KW | 0,08 |
| Strom 57 = 165000. mit | NMP | 91,7 |
| | Wasser | 8,3 |
| | Summe KW | 1 Gew.-ppm |

Temperatur: generell 38°C

Bei den folgenden Angaben zur Beschreibung der Kolonnen werden die Bodenzahlen generell vom Kopf der Kolonne gezählt!

| Betriebsbedingungen für Kolonne 100: | | |
|---|---|---|
| Anzahl theoretischer Böden | = 4 + 50 | (einschließlich Rückwaschzone) |
| KW-Rücklauf auf Boden | = 1 | |
| LM-Zulauf auf Boden | = 5 | |
| KW-Zulauf auf Boden | = 42 | |
| Zusatzstrom Zbc auf Boden | = 42 | |
| Druck auf Boden 1 | = 4,0 bar | |
| Temperatur auf Boden 1 | = 38,5°C | |
| KW-Rücklaufmenge | = 5000 kg/h | |

| Betriebsbedingungen für Kolonne 110: | | |
|---|---|---|
| Anzahl theoretischer Böden | = 4 + 9 | (einschließlich Rückwaschzone) |
| KW-Rücklauf auf Boden | = 1 | |
| Extrakt-Zulauf auf Boden | = 5 | |
| Druck auf Boden 1 | = 1,526 bar | |
| Temperatur auf Boden 1 | = 6,7°C | |
| KW-Rücklaufmenge | = 5000 kg/h | |
| Energiebedarf | = 10361 kW | |

| Betriebsbedingungen für Kolonne 120: | |
|---|---|
| Anzahl theoretischer Böden | = 25 + 23 + 7 (in 2 Kolonnen) |
| KW-Rücklauf auf Boden | = 1 |
| LM-Zulauf auf Boden | = 1 |
| KW-Abzug auf Boden (II) | = 49 (zur Extraktionszone |
| KW-Zulauf auf Boden | = 26 |
| Druck auf Boden 1 | = 4,5 bar |
| Temperatur auf Boden 1 | = 41,5°C |
| KW-Rücklaufmenge | = 2094 kg/h |

| Betriebsbedingungen für Kolonne 130: | | |
|---|---|---|
| Anzahl theoretischer Böden | = 4 + 30 | (einschließlich Rückwaschzone) |
| KW-Rücklauf auf Boden | = 1 | |
| LM-Zulauf auf Boden | = 5 | |
| Druck auf Boden 1 | = 5,0 bar | |
| Temperatur auf Boden 1 | = 45,3 C | |
| KW-Rücklaufmenge | = 2120 kg/h | |

| Betriebsbedingungen für Kolonne 140: | |
|---|---|
| Anzahl theoretischer Böden | =10 |
| Extrakt-Zulauf auf Boden | = 1 |
| KW-Abzug auf Boden | = 6 (zur Kolonne 150) |
| Druck auf Boden 1 | = 1,52 bar |
| Temperatur auf Boden 1 | = 104,6°C |
| Temperatur auf Boden 10 | = 146,1°C |
| KW-Rücklaufmenge | = 2120 kg/h |
| Energiebedarf | = 6773 kW |

| Betriebsbedingungen für Kolonne 150: | |
|---|---|
| Anzahl theoretischer Böden | = 2 |
| Wasser-Zulauf auf Boden | = 1 |
| Druck auf Boden 1 | = 1,52 bar |
| Temperatur auf Boden 1 | = 108°C |

| Betriebsbedingungen für Kolonne 160: | |
|---|---|
| Anzahl theoretischer Böden | = 46 |
| KW-Rücklauf auf Boden | = 1 |
| Extrakt-Zulauf auf Boden | = 16 |
| Druck auf Boden 1 | = 7,0 bar |
| Temperatur auf Boden 1 | = 46,6°C |
| KW-Rücklaufmenge | = 6130 kg/h |
| Energiebedarf | = 761 kW |

| Betriebsbedingungen für Kolonne 170: | |
|---|---|
| Anzahl theoretischer Böden | = 45 |
| KW-Rücklauf auf Boden | = 1 |
| Extrakt-Zulauf auf Boden | = 23 |
| Druck auf Boden 1 | = 4,2 bar |
| Temperatur auf Boden 1 | = 39,3 C |
| KW-Rücklaufmenge | = 11197 kg/h |
| Energiebedarf | = 1671 kW |

Die einzelnen Verfahrensschritte sehen wie folgt aus:

Das üblicherweise flüssige C₄-Kohlenwasserstoffgemisch (Strom 40) wird im Wärmetauscher 240 verdampft und tritt dampfförmig in die Extraktionszone (I), etwa in der Mitte der Kolonne 120, ein. LM über den Strom 42 wird im Gegenstrom den aufsteigenden Gasen entgegengeführt. Dadurch entstehen 2 neue Ströme: ein gasförmiges Produkt (Strom 43) mit im wesentlichen der Hauptmenge an Propan, Propen, Propadien, den Butanen und den Butenen sowie ein Extrakt (Strom 53) mit den im LM gelösten Kohlenwasserstoffen von 1,3-Butadien und den sonstigen Kohlenwasserstoffen, einschließlich der C₅-Kohlenwasserstoffe. Der Gehalt an 1,3-Butadien, eine wichtige Spezifikationsgröße für die Trennforderung der Extraktionszone (I), liegt unter 100 Gew.-ppm.

Zwecks Erniedrigung des Temperaturprofiles in der Kolonne 120 werden 20 Gew.-% des Gasstromes 43 im Wärmetauscher 230 kondensiert.

Über den Strom 51 verläßt ein Seitenstrom die Kolonne 120, der in der Extraktionszone (II), das ist Kolonne 130, mit LM (Strom 46) im Gegenstrom gewaschen wird. Zur Absenkung des Temperaturprofiles und zur gleichzeitigen Verringerung des NMP-Verlustes besitzt die Kolonne einen Rücklauf an Flüssigkohlenwasserstoffen (Strom 50). Die LM-Menge (Strom 46) wird so eingestellt, daß für das spätere 1,3-Reinbutadien die Spezifikationen bezüglich Ethyl- und Vinylacetylen erfüllt sind. Der in den Kopfkreislauf eingebaute Dekanter 320 dient dem partiellen Wasserentzug aus dem Rohbutadien.

Der Extrakt aus der Extraktionszone (I), das ist Strom 53, passiert einen Wärmetauscher 255 und wird anschließend unter Druck im Flash-Behälter 410 partiell desorbiert, wobei 2 Ströme entstehen: ein gasförmiger Anteil (Strom 59), der sofort wieder über Strom 67 in die Kolonne 120 zurückgegeben wird, und ein flüssiger Anteil (Strom 60). Die Temperatur des Stromes 60, der immer noch unter dem erhöhten Flash-Druck des Flash-Behälters 410 steht, wird in einem weiteren Wärmetauscher 260 um 5 C angehoben, bevor er unter Druckreduzierung in die Kolonne 140 entspannt wird.

Der Extrakt, Strom 61, wird in der Kolonne 140 durch zugeführte externe Energie nahezu vollständig von den C₄-Kohlenwasserstoffen desorbiert. Das entstehende Gas, Strom 62, wird im Wärmetauscher 265 auf die bereits früher erwähnten 45 C abgekühlt und im Flash-Behälter 420 in einen geringfügigen Anteil Flüssigphase (Strom 66) und in den Hauptteil Gasphase (Strom 64) aufgeteilt. Der Gasstrom 64 wird im Verdichter 500 komprimiert und nach Vereinigung mit den Strömen 59 und 66 als Strom 67 in den Unterteil der Kolonne 120 zurückgeführt.

Aus der Kolonne 140 wird etwa in der Mitte ein Gasstrom 68 entnommen. Neben Kohlenwasserstoffen enthält er Wasser. Dieser Gasstrom wird in der Kolonne 150 im Gegenstrom mit Kondensat gewaschen, das gasförmige Produkt (Strom 70) im Wärmetauscher 270 abgekühlt und nach Aufteilung in eine Gas- und Flüssigphase (Ströme 72 und 73) als Produkt abgegeben. Der NMP-Gehalt im Strom 70 beträgt rd. 160 Gew.-ppm, was einen NMP-Verlust von 0,19 kg/h an dieser Stelle bedeutet.

Der Weg des nahezu vollständig desorbierten LM aus der Kolonne 140 durchläuft hintereinander die Wärmetauscher 255 (Aufheizen des Extraktes aus der Kolonne 120), 250 (Aufkocher für die Kolonne 170), 240 (Verdampfen des C₄-Kohlenwasserstoffgemisches) und 235 (LM-Schlußkühler zur Einstellung der LM-Temperatur). Damit sind die Extraktionszonen (I) und (III) und die Desorptionszonen (I) und (II) vollständig durchlaufen.

Das Kopfprodukt der Kolonne 120 (Strom 45) sowie der Zusatzstrom Zbc (Strom 11) werden ins untere Drittel der Extraktionszone (II), d. h. Kolonne 100, eingespeist. Zusammen mit dem nach Wärmetausch im Wärmetauscher 215 und anschließender Flash-Entspannung im Flash-Behälter 400 entstehenden Gasstrom werden die Gase im Gegenstrom zum LM (Ströme 12 und 57) geführt. Dabei entsteht eine hoch reine Butane-Fraktion (Strom 13) mit nur 0,43 Gew.-% Butene. Wie das Kopfprodukt der Kolonne 130 wird auch der Gasstrom 13 kondensiert (Wärmetauscher 200) und anschließend im Dekanter 300 teilweise von Wasser befreit, ehe er als Flüssigkohlenwasserstoffrücklauf (Strom 16) wieder der Kolonne 100 zugeführt oder als Butanefraktion (c), d. h. Strom 17, als Produkt abgegeben wird.

Der bereits durch Wärmetausch erwärmte Extrakt aus der Extraktionszone (II), d. h. Strom 20, wird nach erneutem indirekten Wärmeaustausch im Wärmetauscher 220 nach Druckreduzierung der Desorptionszone (III), d. h. Kolonne 110, zugeführt. Durch Eintrag externer Wärme wird dort der Extrakt weitgehendst von den C₄-Kohlenwasserstoffen befreit. Die so entstehende Butenefraktion (b), Strom 23, ist ebenfalls hoch rein und enthält nur 1,85 Gew.-% Butane. Nach Kondensation im Wärmetauscher 225 und teilweiser Wasserabtrennung im Dekanter 225 wird die Butenefraktion zum Teil in flüssiger Form als Rücklauf wieder der Kolonne 110 zugeführt (Strom 26) bzw. als Produkt (Strom 27) abgegeben.

Der Wärmetauscher 225 benötigt wegen der niedrigen Kondensationstemperatur der Butenefraktion ein Kältemittel. Es ist nicht möglich, das Druckniveau in der Kolonne 110 anzuheben: Zum einen würde die Sumpftemperatur der Kolonne 110 über die Grenze von 150 C hinausgehen, was einer unerlaubten thermischen Belastung des LM gleich käme, und zum anderen würde die Desorption der Kohlenwasserstoffe erschwert, was nur mit einem zusätzlichen Eintrag externer Energie zu kompensieren wäre.

Das in der Kolonne 110 desorbierte LM durchläuft nacheinander die Wärmetauscher 220 (Extraktvorheizung), 215 (Temperaturerhöhung des Extraktes zwecks Druck-Flashs), 210 (Verdampfer des Zusatzstromes 10) und 205 (Schlußkühler zur Einstellung der LM-Temperatur). Wegen der vielfältigen Aufgaben sind gewisse Temperaturniveaus vorgegeben, weswegen der Kohlenwasserstoffgehalt des Stromes 28 nicht beliebig hoch sein darf. Im Beispielsfalle beträgt die Konzentration 800 Gew.-ppm. Damit sind auch die Absorptionsund Desorptionszonen (III) vollständig durchlaufen.

Es verbleibt nur noch der Bereich mit den Destillationskolonnen 160 und 170 zum endgültigen Einstellen der Spezifikationen der 1,3-Butadienfraktion (a). Die Einspeisung des Rohbutadiens (Strom 80) erfolgt im oberen Drittel der Kolonne 160. Das gasförmige Kopfprodukt (Strom 81) wird kondensiert und nach partieller Wasserabscheidung im Dekanter 330 sowohl als Flüssigkohlenwasserstoffrücklauf (Strom 84) in die Kolonne 160 zurückgegeben als auch als Produkt (Strom 83) abgegeben. Zu beachten ist, daß Strom 81 aus Sicherheitsgründen eine gewisse Propinkonzentration nicht überschreiten darf. Diese Grenzkonzentration ist druckabhängig. Bei einem Kopfdruck von 7 bar liegt dieser Wert bei 50 Vol.-%.

Der nahezu wasserfreie Sumpfabzug der Kolonne 160 wird etwa der Mitte der Kolonne 170 zugeführt. Dort wird das Gemisch in eine hoch reine 1,3-Butadienfraktion (Strom 89) und ein Sumpfprodukt (Strom 91) aufgetrennt, wobei Strom 91 ein Gemisch hauptsächlich aus den Kohlenwasserstoffen cis-Buten-2, 1,3-Butadien, 1,2-Butadien und C₅-Kohlenwasserstoffen darstellt. Durch Vorgabe der 1,3-Butadienkonzentration kann man die Ausbeute an 1,3-Butadien beeinflussen. Im vorliegenden Beispiel beträgt diese 25 Gew.-%. Die Produktspezifikation der Fraktion (a), d. h. das Reinbutadien, lautet:

| | |
|---|---|
| 1,3-Butadien | = 99,6 Gew.-% |
| Summe Butene | = 0,4 Gew.-% |
| Propin | = 10 Gew.-ppm |
| 1,2-Butadien | = 50 Gew.-ppm |
| Summe C₄-Acetylene | < 5 Gew.-ppm |
| Summe C₅-KW | < 5 Gew.-ppm |

Abschließend sind summarisch für Kondensatoren und Wärmetauscher die getauschten Energiemengen [kW] aufgelistet:

| | |
|---|---|
| Wärmetauscher | 200 : 785 |
| Wärmetauscher | 205 : 8991 |
| Wärmetauscher | 210 : 482 |
| Wärmetauscher | 215 : 3127 |
| Wärmetauscher | 220 : 4000 |
| Wärmetauscher | 225 : 1679 |
| Wärmetauscher | 230 : 230 |
| Wärmetauscher | 235 : 2945 |
| Wärmetauscher | 240 : 1772 |
| Wärmetauscher | 245 : 857 |
| Wärmetauscher | 250 : 1671 |
| Wärmetauscher | 255 : 7264 |
| Wärmetauscher | 260 : 744 |
| Wärmetauscher | 265 : 1729 |
| Wärmetauscher | 270 : 572 |
| Wärmetauscher | 275 : 693 |
| Wärmetauscher | 280 : 1757 |

## Patentansprüche

1. Verfahren zur Trennung eines C₄-Kohlenwasseretoffgemisches enthaltend im wesentlichen 1,3-Butadien, Butene, Butane, und sonstige C₄-Kohlenwasserstoffe, in mindestens 4 Fraktionen, wobei
a) die Fraktion (a) im wesentlichen aus 1,3-Butadien,
b) die Fraktion (b) im wesentlichen aus Butenen
c) die Fraktion (c) im wesentlichen auß Butanen und
d) eine oder mehrere Fraktionen (d), die im wesentlichen aus 1,3-Butadien und den sonstigen C₄-Kohlenwasserstoffen bestehen,
durch Extraktivdestillation mittels N-Methyl-2-pyrrolidinon oder einer wässerigen Lösung von N-Methyl-2-pyrrolidinon (NMP),
wobei man
1. das gasförmige C₄-Kohlenwasserstoffgemisch zunächst mit NMP in einer Extraktionszone (I) in Kontakt bringt, wobei das Mengenverhältnis NMP zu C₄-Kohlenwasserstoffgemisch 5 : 1 bis 20 : 1 beträgt, das 1,3-Butadien und die sonstigen C₄-Kohlenwasserstoffe vom NMP im wesentlichen vollständig absorbiert werden, die Butene und Butane jedoch im wesentlichen in der Gasphase verbleiben;
2. die nicht-absorbierten Butene und Butane (Gasstrom bc) und die in Schritt 1 gebildete Extraktionslösung (Extraktionslösung ad) getrennt aus dar Extraktionszone (I) entfernt;
3. die Extraktionslösung (ad) in eine Desorptionszone (I) mit gegenüber der Extraktionszone (I) vermindertem Druck und/oder erhöhter Temperatur überführt und dabei aus der Extraktionslösung (ad) 1,3-Butadien desorbiert, wobei der Hauptteil der sonstigen C₄-Kohlenwasserstoffe in der flüssigen Phase verbleibt;
4. die in Stufe 3 gebildete Extraktionslösung (Extraktionslösung d) und das desorbierte 1,3-Butadien (Fraktion a) getrennt aus der Desorptionszone (I) entfernt und gegebenenfalls einen Teil der Fraktion (a) in die Extraktionszone I zurückführt;
5. die Extraktionslösung (d) in eine zweite Desorptionszone (II) mit gegenüber der Desorptionszone (I) vermindertem Druck und/oder erhöhter Temperatur und mit einem Druckund/oder Temperaturgradienten überführt und dabei aus der Extraktionslösung (d) noch darin verbliebenes 1,3-Butadien und die sonstigen C₄-Kohlenwasserstoffe mindestens als 2 getrennte Fraktionen (d) fraktioniert desorbiert, wobei in mindestens einer der Fraktionen (Fraktionen d) der Anteil der sonstigen C₄-Kohlenwasserstoffe um das mindestens 10fache höher ist als in der Extraktionslösung (d), bezogen auf den Anteil aller C₄-Kohlenwasserstoffe, und in mindestens einer der Fraktionen (Fraktionen dR) der Anteil der sonstigen C₄-Kohlenwasserstoffe entsprechend niedriger ist als in den Fraktionen (d), bezogen auf den Anteil aller C₄-Kohlenwasserstoffe,
6. das in der Desorptionszone (II) gebildete NMP, das im wesentlichen frei ist von C₄-Kohlenwasserstoffen, und die Fraktionen (d) und (dR) getrennt aus der Desorptionszone II entfernt und eine oder mehrere Fraktionen (dR) in die Desorptionszone (I) zurückführt,
7. den Gasstrom (bc) zunächst mit NMP in einer Extraktionszone (II) in Kontakt bringt, wobei die Butene vom NMP im wesentlichen vollständig absorbiert werden, die Butane jedoch im wesentlichen in der Gasphase verbleiben,
8. die nicht-absorbierten Butane (Fraktion c) und die in Schritt 1 gebildete Extraktionslösung (Extraktionslosung b) aus der Extraktionszone (II) entfernt;
9. die Extraktionslösung (b) in eine Desorptionszone (III) mit gegenüber der Extraktionszone (II) vermindertem Druck und/oder erhöhter Temperatur überführt und dabei aus der Extraktionslösung (b) die Butene desorbiert;
10. das in Schritt 9 gebildete NMP, das im wesentlichen frei von C₄-Kohlenwasserstoffen ist, und die desorbierten Butene (Fraktion b) aus der Desorptionszone (III) entfernt;
11. das in Schritt 9 gabildete NMP in die Extraktionszonen I und II zurückführt.
12. einen Teil der 1,3-Butadienfraktion (a), die man aus der Desorptionszone (I) entfernt, in die Extraktionszone (I) zurückfahrt (Fraktion a1) und den anderen Teil (Fraktion a2) nochmals in eine Extraktionszone (III) mit NMP in Kontakt bringt, das gemäß Schritt 6 des Anspruchs 1 zurückgewonnen wurde, wobei das Verhältnis an NMP zur Rohbutadienfraktion (a2) 1 : 3 bis 1 : 7 beträgt und ein Teil der Fraktion (a2) und der überwiegende Teil an sonstigen C₄-Kohlenwasserstoffen, die noch als Verunreinigung in der Fraktion (a2) enthalten sind, vom NMP absorbiert werden (Extraktionslösung ax) ; und
13. den nicht absorbierten Teil der Fraktion (a2) (Fraktion a3) aus der Extraktionszone getrennt entfernt und die Extraktionslösung (ax) in die Extraktionszone (I) zurückführt.

2. Verfahren nach Anspruch 1, **dadurch gekennzeichnet, daß** man das NMP und das C₄-Kohlenwasserstoffgemisch in einem Massenverhältnis 5:1 bis 20:1 in die Extraktionszone (I) einspeist.

3. Verfahren nach Anspruch 1 oder 2, **dadurch gekennzeichnet, daß** das C₄-Kohlenwasseretoffgemisch
- 10 bis 80 Gew.-% 1,3-Butadien,
- 10 bis 60 Gew.-% Butene;
- 5 bis 40 Gew.-% Butane; und
- 0,1 bis 5 Gew.-% sonstige C₄-Kohlenwasserstoffe enthält.

4. Verfahren nach den Ansprüchen 1 bis 3, **dadurch gekennzeichnet, daß** die Extraktionszone (I) in Form von 2 miteinander verbundener Kolonnen ausgeführt ist,

5. Verfahren nach den Ansprüchen 1 bis 4, **dadurch gekennzeichnet, daß** man die Extraktionszonen (I), (II), und (III) in Form von Kolonnen ausführt, und man durch diese die Gasströme im Gegenstrom zum NMP leitet.

6. Verfahren nach den Ansprüchen 1 bis 5, **dadurch gekennzeichnet, daß** man die in Schritt 3 und 5 des Anspruchs 1 beschriebene Desorption dadurch bewirkt, daß man die Temperatur in der Desorptionszone (I) gegenüber der in der Extraktionszone (I) und die Temperatur in der Desorptionszone (II) gegenüber der in der Desorptionszone (I) erhöht, indem man die Wärme des NMP, das man gemäß schritt 11 von Anspruch 1 aus der Desorptionszone (II) entfernt hat, durch indirekten Wärmeaustausch in einer Wärmeaustauschzone, der Desorptionszone (I) zuführt.

7. Verfahren nach den Ansprüchen 1 bis 6, **dadurch gekennzeichnet, daß** man zum in Schritt 1 gebildeten Gasstrom einen weiteren Kohlenwasserstoffstrom (Zusatzstrom zbc) hinzufügt, wobei letzterer im wesentlichen aus einem Gemisch von Butanen und Butenen besteht.

8. Verfahren nach den Ansprüchen 1 bis 7, **dadurch gekennzeichnet, daß** man die Temperaturerhöhung in den Desorptionszonen (II) und (III) zwecks vollständiger Desorption des NMP von Kohlenwasserstoffen durch indirekten Wärmeaustausch mit Dampf vornimmt.

9. Verfahren nach den Ansprüchen 1 bis 8, **dadurch gekennzeichnet, daß** das zur Trennung der Fraktionen (b) und (c) nötige Rückgas in der Extraktionszone (II) durch partielle Desorption der Extraktionslösung (Eb) gewonnen wird, wobei die nötige Temperaturerhöhung durch indirekten Wärmetausch aus dem von C₄-Kohlenwasserstoffen befreitem NMP der Desorptionszone (III) stammt.

## Claims

1. A process for separating a C₄-hydrocarbon mixture essentially containing 1,3-butadiene, butenes, butanes and other C₄-hydrocarbons into at least 4 fractions,
a) the fraction (a) essentially comprising 1,3-butadiene,
b) the fraction (b) essentially comprising butenes,
c) the fraction (c) essentially comprising butanes and
d) one or more fractions (d) essentially comprising 1,3-butadiene and the other C₄-hydrocarbons,
by extractive distillation by means of N-methyl-2-pyrrolidinone or an aqueous solution of N-methyl-2-pyrrolidinone (NMP),
wherein
1. the gaseous C₄-hydrocarbon mixture is first brought into contact with NMP in an extraction zone (I), the ratio of NMP to C₄-hydrocarbon mixture being from 5:1 to 20:1, the 1,3-butadiene and the other C₄-hydrocarbons being essentially completely absorbed by the NMP but the butenes and butanes remaining essentially in the gas phase;
2. the unabsorbed butenes and butanes (gas stream bc) and the extraction solution formed in step 1 (extraction solution ad) are removed from the extraction zone (I);
3. the extraction solution (ad) is transferred to a desorption zone (I) at a lower pressure and/or higher temperature than the extraction zone (I) and 1,3-butadiene is desorbed from the extraction solution (ad), the main part of the other C₄-hydrocarbons remaining in the liquid phase;
4. the extraction solution formed in stage 3 (extraction solution d) and the desorbed 1,3-butadiene (fraction a) are removed separately from the desorption zone (I) and, if required, a part of the fraction (a) is returned to the extraction zone I;
5. the extraction solution (d) is transferred to a second desorption zone (II) at a lower pressure and/or higher temperature than the desorption zone (I) and having a pressure and/or temperature gradient, and the other C₄-hydrocarbons and the 1,3-butadiene still remaining therein are fractionally desorbed from the extraction solution (d) as at least two separate fractions (d), with the content of the other C₄-hydrocarbons being at least 10 times higher in at least one of the fractions (fractions d) than in the extraction solution (d), based on the content of all C₄-hydrocarbons, and the content of the other C₄-hydrocarbons being correspondingly lower in at least one of the fractions (fractions dR) than in the fractions (d), based on the content of all C₄-hydrocarbons,
6. the NMP, formed in the desorption zone (II) and essentially free of C₄-hydrocarbons, and the fractions (d) and (dR) are removed separately from the desorption zone II, and one or more fractions (dR) are returned to the desorption zone (I),
7. the gas stream (bc) is first brought into contact with NMP in an extraction zone (II), the butenes being essentially completely absorbed by the NMP but the butanes remaining essentially in the gas phase;
8. the unabsorbed butanes (fraction c) and the extraction solution formed in step 1 (extraction solution b) are removed from the extraction zone (II);
9. the extraction solution (b) is transferred to a desorption zone (III) at a lower pressure and/or higher temperature than the extraction zone (II) and the butenes are desorbed from the extraction solution (b);
10. the NMP, formed in step 9 and essentially free of C₄-hydrocarbons, and the desorbed butenes (fraction b) are removed from the desorption zone (III);
11. the NMP formed in step 9 is recycled to the extraction zones I and II,
12. a part of the 1,3-butadiene fraction (a) which is removed from the desorption zone (I) is returned to the extraction zone (I) (fraction a1) and the other part (fraction a2) is again brought into contact, in an extraction zone (III), with NMP which was recovered according to step 6 of claim 1, the ratio of NMP to the crude butadiene fraction (a2) being from 1:3 to 1:7 and a part of the fraction (a2) and the predominant part of other C₄-hydrocarbons still contained as impurity in the fraction (a2) being absorbed by the NMP (extraction solution ax); and
13. the unabsorbed part of the fraction (a2) (fraction a3) is removed separately from the extraction zone, and the extraction solution (ax) is returned to the extraction zone (I).

2. A process as claimed in claim 1, wherein the NMP and the C₄-hydrocarbon mixture are fed into the extraction zone (I) in a ratio by weight of from 5:1 to 20:1.

3. A process as claimed in claim 1 or 2, wherein the C₄-hydrocarbon mixture contains
- from 10 to 80% by weight of 1,3-butadiene;
- from 10 to 60% by weight of butenes;
- from 5 to 40% by weight of butanes; and
- from 0.1 to 5% by weight of other C₄-hydrocarbons.

4. A process as claimed in any of claims 1 to 3, wherein the extraction zone (I) is in the form of 2 columns connected to one another.

5. A process as claimed in any of claims 1 to 4, wherein the extraction zones (I), (II) and (III) are in the form of columns and the gas streams are passed countercurrently to the NMP through said columns.

6. A process as claimed in any of claims 1 to 5, wherein the desorption described in steps 3 and 5 of claim 1 is effected by increasing the temperature in the desorption zone (I) relative to that in the extraction zone (I) and increasing the temperature in the desorption zone (II) relative to that in the desorption zone (I) by supplying the heat of the NMP, which has been removed according to step 11 of claim 1 from the desorption zone (II), to the desorption zone (I) by indirect heat exchange in a heat exchange zone.

7. A process as claimed in any of claims 1 to 6, wherein another hydrocarbon stream (added stream Zbc), the latter essentially consisting of a mixture of butanes and butenes, is added to the gas stream formed in step 1.

8. A process as claimed in any of claims 1 to 7, wherein the temperature is increased in the desorption zones (II) and (III) for the purpose of complete desorption of the NMP from hydrocarbons by indirect heat exchange with steam.

9. A process as claimed in any of claims 1 to 8, wherein the back gas required for separating the fractions (b) and (c) is obtained in the extraction zone (II) by partial desorption of the extraction solution (Eb), the required increase in temperature originating, by indirect heat exchange, from the NMP freed of C₄-hydrocarbons in the desorption zone (III).

## Revendications

1. Procédé pour séparer un mélange d'hydrocarbures en C₄ contenant essentiellement du 1,3-butadiène, des butènes, des butanes, et d'autres hydrocarbures en C₄, en au moins 4 fractions, où
a) la fraction (a) est essentiellement composée de 1,3-butadiène,
b) la fraction (b) est essentiellement composée de butènes,
c) la fraction (c) est essentiellement composée de butanes et
d) une ou plusieurs fractions (d) sont essentiellement composées de 1,3-butadiène et des autres hydrocarbures en C₄,
par une distillation extractive au moyen de la N-méthyl-2-pyrrolidinone ou d'une solution aqueuse de N-méthyl-2-pyrrolidinone (NMP),
procédé dans lequel
1) on met d'abord en contact, dans une zone d'extraction (I), le mélange d'hydrocarbures en C₄ gazeux avec NMP, le rapport massique de NMP au mélange d'hydrocarbures en C₄ allant de 5:1 à 20:1, où le 1,3-butadiène et les autres hydrocarbures en C₄ sont presque entièrement absorbés par NMP, les butènes et les butanes restant toutefois essentiellement dans la phase gazeuse;
2) on élimine, de manière séparée, de la zone d'extraction (I), les butènes et les butanes non absorbés (courant de gaz bc) et la solution d'extraction formée dans l'étape I (solution d'extraction ad);
3) on transfère la solution d'extraction (ad) dans une zone de désorption (I) présentant une pression inférieure et/ou une température supérieure à la zone d'extraction (I), pour désorber ainsi 1,3-butadiène à partir de la solution d'extraction (ad), la majeure partie des autres hydrocarbures en C₄ restant dans la phase liquide;
4) on élimine, de manière séparée, de la zone de désorption (I) la solution d'extraction formée dans l'étape 3 (solution d'extraction d) et le 1,3-butadiène désorbé (fraction a), et on recycle éventuellement une partie de la fraction (a) dans la zone d'extraction I;
5) on transfère la solution d'extraction (d) dans une deuxième zone de désorption (II) présentant une pression inférieure et/ou une température supérieure à la zone de désorption (I) et présentant un gradient de pression et/ou de température, pour désorber ainsi de manière fractionnée, à partir de la solution d'extraction (d), le 1,3-butadiène restant ainsi que les autres hydrocarbures en C₄, sous forme d'au moins deux fractions (d) séparées, la proportion des autres hydrocarbures en C₄ étant, dans au moins une des fractions (fractions d), au moins 10 fois plus élevée que dans la solution d'extraction (d) et par rapport à la proportion de la totalité des hydrocarbures en C₄, et la proportion des autres hydrocarbures en C₄ étant, dans au moins une des fractions (fractions dR), en conséquence moins élevée par rapport aux fractions (d) et par rapport à la proportion de la totalité des hydrocarbures en C₄;
6) on élimine, de manière séparée, de la zone de désorption II la NMP, formée dans la zone de désorption (II), et essentiellement exempte d'hydrocarbures en C₄, et les fractions (d) et (dR), et on recycle une ou plusieurs fractions (dR) dans la zone de désorption (I);
7) on met en contact d'abord le courant de gaz (bc) avec NMP dans une zone d'extraction (II), où les butènes sont presque entièrement absorbés par NMP, les butanes restant essentiellement dans la phase gazeuse;
8) on élimine de la zone d'extraction (II) les butanes non absorbés (fraction c) et la solution d'extraction formée dans l'étape 1 (solution d'extraction b);
9) on transfère la solution d'extraction (b) dans une zone de désorption (III) présentant une pression inférieure et/ou une température supérieure à la zone d'extraction (II) pour désorber ainsi les butènes à partir de la solution d'extraction (b);
10) on élimine de la zone de désorption (III) la NMP, formée dans l'étape 9 et essentiellement exempte d'hydrocarbures en C₄, et les butènes désorbés (fraction b);
11) on recycle, dans les zones d'extraction I et II, la NMP formée dans l'étape 9;
12) on recycle, dans la zone d'extraction (I), une partie de la fraction de 1,3-butadiène (a) (fraction a1) que l'on a éliminée de la zone de désorption (I), et on met de nouveau en contact, dans une zone d'extraction (III), l'autre partie (fraction a2) avec NMP que l'on a obtenue selon l'étape 6 de la revendication 1, le rapport de NMP à la fraction de butadiène brut (a2) allant de 1:3 à 1:7, et une partie de la fraction (a2) et la majeure partie des autres hydrocarbures en C₄, encore présents sous forme d'impuretés dans la fraction (a2), étant absorbées par NMP (solution d'extraction ax); et
13) on élimine, de manière séparée, de la zone d'extraction, la partie non absorbée de la fraction (a2) (fraction a3) et on recycle dans la zone d'extraction (I) la solution d'extraction (ax).

2. Procédé selon la revendication 1, **caractérisé en ce que** l'on charge, dans la zone d'extraction (I), NMP et le mélange d'hydrocarbures en C₄, dans un rapport massique allant de 5:1 à 20:1.

3. Procédé selon la revendication 1 ou 2, **caractérisé en ce que** le mélange d'hydrocarbures en C₄ contient
- le 1,3-butadiène à raison de 10% à 80% en poids,
- des butènes à raison de 10% à 60% en poids,
- des butanes à raison de 5% à 40% en poids, et
- d'autres hydrocarbures en C₄ à raison de 0,1% à 5% en poids.

4. Procédé selon les revendications 1 à 3, **caractérisé en ce que** l'on construit la zone d'extraction (I) sous la forme de 2 colonnes qui sont reliées l'une à l'autre.

5. Procédé selon les revendications 1 à 4, **caractérisé en ce que** l'on construit les zones d'extraction (I), (II) et (III) sous la forme de colonnes à travers lesquelles on amène les courants de gaz à contrecourant par rapport à NMP.

6. Procédé selon les revendications 1 à 5, **caractérisé en ce que** l'on réalise la désorption décrite dans les étapes 3 et 5 de la revendication 1 en élevant la température dans la zone de désorption (I) par rapport à celle de la zone d'extraction (I), et en élevant la température dans la zone de désorption (II) par rapport à celle de la zone de désorption (I), en amenant la chaleur de NMP, que l'on a éliminé de la zone de désorption (II) selon l'étape 11 de la revendication 1, dans la zone de désorption (I) au moyen d'un échange thermique indirect dans une zone d'échange thermique.

7. Procédé selon les revendications 1 à 6, **caractérisé en ce que** l'on ajoute au courant de gaz formé dans l'étape 1, un courant d'hydrocarbures supplémentaire (courant supplémentaire Zbc), ce dernier étant essentiellement composé d'un mélange de butanes et de butènes.

8. Procédé selon les revendications 1 à 7, **caractérisé en ce que** l'on élève la température dans les zones de désorption (II) et (III) au moyen d'un échange thermique indirect avec de la vapeur en vue de la désorption complète du NMP des hydrocarbures.

9. Procédé selon les revendications 1 à 8, **caractérisé en ce que** l'on produit, dans la zone d'extraction (II), le gaz de recyclage qui est nécessaire pour la séparation des fractions (b) et (c), au moyen d'une désorption partielle de la solution d'extraction (Eb), l'élévation de température nécessaire, par échange thermique indirect, provenant du NMP, libéré des hydrocarbures en C₄, de la zone de désorption (III).
